# EUROPÄISCHE PATENTSCHRIFT

(11) **EP 4 611 861 B1**
(45) Veröffentlichungstag und Bekanntmachung des Hinweises auf die Patenterteilung: **25.11.2026**
(21) Anmeldenummer: 23798295.4
(22) Anmeldetag: 25.10.2023
(51) Int. Cl.: A24F 40/40, A61M 15/00, A61M 11/04, A61M 15/06, A24F 40/20

(54) **VAPORIZER ZUR VERDAMPFUNG EINES INHALATIONSMEDIUMS**
VAPORISER FOR VAPORISING AN INHALATION MEDIUM
VAPORISATEUR POUR LA VAPORISATION D'UN MILIEU D'INHALATION

(30) Priorität: 04.11.2022 CH 13172022
(43) Veröffentlichungstag der Anmeldung: 10.09.2025
(73) Patentinhaber: SKIETTA AG, 6438 Ibach (CH)
(72) Erfinder: QUERCI, Lorenzo, 6438 Ibach (CH)
(74) Vertreter: Schirbach, Marcel
(86) Internationale Anmeldenummer: PCT/IB2023/060766
(87) Internationale Veröffentlichungsnummer: WO 2024/095105

(56) Entgegenhaltungen:
- WO-A1-2013/156339
- WO-A1-2021/119055
- AT-B- 396 872
- DE-B3- 102016 005 345
- US-A1- 2019 254 345

## Beschreibung

### Technisches Gebiet

Die vorliegende Erfindung fällt in das technische Gebiet des täglichen Lebensbedarfs; sie betrifft einen Vaporizer zur Verdampfung eines Inhalationsmediums gemäss dem Oberbegriff des Anspruchs 1.

### Stand der Technik, technologischer Hintergrund der Erfindung

Cannabis oder pflanzliches Marihuana und der daraus erzeugte Dampf haben sich seit langem als medizinisch nützlich erwiesen, z. B. bei der Behandlung von Krebs, Glaukom, Krampfanfällen, Multipler Sklerose, Epilepsie, HIV und anderen Krankheiten. Eine Person, die mit Cannabis behandelt wird, kann eine Appetitanregung, Schmerzlinderung, Entspannung, Verringerung von Entzündungen und/oder andere Vorteile erfahren. Diese Wirkungen sind zum großen Teil auf Cannabinoide zurückzuführen, chemische Verbindungen, die in Cannabis vorkommen und auf das Cannabinoid-Rezeptorsystem des Gehirns wirken. Das Cannabinoid-Rezeptorsystem ist an einer Reihe von physiologischen Prozessen beteiligt, darunter Appetit, Schmerzempfinden, Stimmung und Gedächtnis. Endocannabinoide sind körpereigene Substanzen (z. B. neuromodulatorische Lipide), die Cannabinoidrezeptoren aktivieren. Es kann jedoch wünschenswert sein, dass die Menge der Endocannabinoide im Körper erhöht wird. Daher können die in Cannabis enthaltenen Cannabinoide in einigen Fällen dazu dienen, das Vorhandensein von Endocannabinoiden wiederherzustellen oder zu verstärken, die ansonsten im System fehlen könnten.

In bestimmten Regionen der Vereinigten Staaten können Ärzte ihren Patienten die Verwendung von Cannabis verschreiben. Allerdings sind die Möglichkeiten der Cannabisabgabestellen, gleichbleibende Mengen und Arten von Cannabis mit einem bestimmten Grad an therapeutischer Qualität zuverlässig abzugeben, begrenzt. Beim Konsum von medizinischem Cannabis müssen die Patienten beispielsweise oft lose Cannabisknospen oder -blätter erwerben, die in einem offenen Beutel aufbewahrt werden, zusammen mit Stöcken, Samen und anderen nicht konsumierbaren Rückständen. Wenn Cannabis auf diese Weise beschafft wird, können Sicherheitsbedenken entstehen, da die Gefahr einer Verunreinigung, Manipulation usw. besteht. Solcher Cannabis kann auch eine relativ kurze Haltbarkeitsdauer haben und vor dem Gebrauch umständlich zu handhaben sein, z. B. durch das Trennen von Stöcken, Samen und Resten oder durch das Zerkleinern und Verpacken des Cannabis in Behälter für den späteren Konsum.

Werden Pflanzenteile der weiblichen Hanfpflanze (meist Cannabis sativa, Cannabis indica oder deren Kreuzungen) konsumiert, können sie eine berauschende Wirkung hervorrufen.

Umgangssprachliche Namen für diese Pflanzenprodukte sind etwa Gras, Weed oder Marihuana (für die weiblichen Blüten) und Haschisch (für das daraus gewonnene und gepresste Harz). Auch Extrakte wie Haschischöl werden als Rauschmittel genutzt. Besitz und Weitergabe solcher Cannabisprodukte sind in den meisten Ländern verboten. Das ist auch in Deutschland, Österreich und der Schweiz der Fall, wo Cannabis, wie auch in vielen anderen Ländern dieser Erde mit einer Cannabis-Prohibition, die am häufigsten konsumierte illegale Rauschdroge ist.

Es eignen sich jedoch nicht alle Cannabissorten als Rauschmittel. Denn z. B. Nutzhanfsorten (aber auch manche medizinische Cannabissorten) enthalten den dazu notwendigen Wirkstoff Tetrahydrocannabinol (THC) nur in äußerst geringen, nicht spürbar wirkenden Mengen. THC beeinflusst das Zentralnervensystem des Menschen und ist vorrangig für die muskelentspannende, beruhigende und übelkeitsunterdrückende Wirkung verantwortlich. Weltweit nutzen im Jahr 2019 nach konservativen Schätzungen ca. 200 Millionen Menschen (≈ 4 % der Weltbevölkerung) Cannabis als Rauschmittel.

Die Cannabispflanze hat eine lange Geschichte des menschlichen Gebrauchs. Viele alte Kulturen züchten die Pflanze als pflanzliche Medizin und atmeten den bei der Verbrennung erzeugten Rauch beispielsweise zu zeremoniellen Anlässen ein. Verbrannte Cannabissamen wurden bereits seit 500 v.Chr. in den Gräbern von Schamanen in China und Sibirien gefunden. Heutzutage verwenden viele Konsumenten immer noch getrocknete Blätter, Öle, Extrakt und andere Arten dieser Pflanze für Freizeit- und medizinische Zwecke. Die überwiegende Mehrheit konsumiert Cannabis, indem sie handgerollte Zigaretten -auch Joints genannt- raucht. Die Verbrennung von Cannabis produziert jedoch Hunderte von chemischen Verbindungen, von denen sich einige als krebserregend erwiesen haben, sowie Teer, welcher von Tabakzigaretten erzeugt wird.

Es gibt jedoch Hinweise darauf, dass der Konsum von Cannabis mit einem Vaporizer eine vergleichsweise zum Joint sicherere Methode sein könnte. In jüngster Zeit hat sich dieser Begriff Vaporizer für Geräte zur Heißextraktion von Wirkstoffen zur inhalativen Aufnahme von Cannabisprodukten durchgesetzt. Dabei handelt es sich ausschließlich um Geräte für Trockensubstanzen, wie z. B. Medizinaldrogen. Ein Vaporizer ist damit eindeutig abgegrenzt gegenüber einer E-Zigarette, die im Wesentlichen für Flüssigkeiten geeignet ist, die meist aromatisiert sind und oft auch Nicotin enthalten können.

Die Vaporizer, oder auch Verdampfer genannt, setzen Cannabisprodukte einer Wärme aus, die dann die in der Pflanze vorhandenen Cannabinoide in Dampf umwandelt. Dieser Dampf wird dann eingeatmet. Das inhalative Aerosol bewahrt dabei den komplexen Geschmack jedes Stammes und die vom Benutzer gewünschte Wirkung. Aus der Internationalen Publikation WO 2015/116934 A1 ist ein herkömmlicher Vaporizer bekannt, der im Wesentlichen einen zweiteiligen Aufbau aufweist, wobei eine unten angeordnete Basis von einem darauf plazierbaren Kanister zwecks Befüllung des Vaporizers mit einer Kapsel voneinander trennbar sind. Für die Handhabung dieses Vaporizers müssen für die Befüllung diese zwei Einheiten, namentlich Basis und Kanister, voneinander getrennt werden, damit eine Öffnung zum Befüllen des Vaporizers freigegeben wird und mit einer Kapsel -gefüllt mit einem Inhalationsmedium- eingesetzt werden kann; anschliessend ist der Kanister wieder auf der Basis mit der Kapsel zu platzieren und zu verschliessen, und zwar derart, dass nicht unkontrolliert Dampf an der Trennlinie Basis/Kanister entweichen kann.

Vor diesem Hintergrund kann es als aufwendig angesehen werden, den Vaporizer des Standes der Technik handzuhaben, respektive zu befüllen, was zudem mit einem entsprechenden Zeitaufwand verbunden ist.

Die folgenden Vaporizer sind aus dem Stand der Technik bekannt, nämlich US 2019/254345 A1, DE 10 2016 005345 B3, WO 2013/156339 A1, AT 396 872 B und WO 2021/119055 Al.

### Darstellung der Erfindung

Es ist deshalb Aufgabe der vorliegenden Erfindung, einen Vaporizer dahingehend weiterzuentwickeln, dass eine vereinfachte und raschere Handhabung bei der Befüllung dieses Vaporizers mit einer Kapsel ermöglicht wird und somit der Genuss von Inhalationsmedien deutlich erleichtert wird.

Die der Erfindung für den Vaporizer zur Verdampfung eines Inhalationsmediums zugrundeliegende Aufgabe wird gelöst durch die Merkmale des Anspruchs 1; die diesen Erfindungsgedanken weiterbildenden Merkmale sind jeweils Gegenstand der Unteransprüche.

Der Kern der vorliegenden Offenbarung ist darin zu sehen, dass der neue Vaporizer zur Verdampfung eines Inhalationsmediums ein Gehäuse mit einer Gehäuseabdeckung in einem oberen Bereich des Gehäuses und eine Zuluftöffnung in einem unteren Bereich des Gehäuses und einen Dampfauslass aufweist, wobei der Vaporizer ein Trägerelement mit einem daran angeordneten Kapselhalter aufweist, welches Trägerelement zumindest abschnittsweise einen kreisbogenförmigen Querschnitt aufweist und wobei der Kapselhalter mit diesem Trägerelement an einem Führungselement entlang einer kreisbogenförmigen Bewegungsrichtung verschiebbar angeordnet ist und der Kapselhalter in einer geöffneten Position des Trägerelements bedienbar, respektive mit dem Inhalationsmedium befüllbar, aus dem Vaporizer herausragt. Der offenbarungsgemässe Vaporizer zeichnet sich durch seine besonders einfache und schnelle Handhabung beim Befüllen mit einer Kapsel aus, wobei der Kapselhalter am Trägerelement aus dem Vaporizer entlang eines Kreisbogens derart ausfahrbar ist, dass sich eine besonders grosse Öffnung und ein demzufolge einfacher Zugang zum zu befüllenden Kapselhalter ergibt.

Bei einer vorteilhaften Ausführungsform des offenbarungsgemässen Vaporizers ist vorgesehen, dass das Führungselement ebenfalls einen zumindest abschnittsweise kreisbogenförmigen Querschnitt aufweist, wobei der zumindest abschnittsweise kreisbogenförmige Querschnitt des Führungselements einen identischen Radius wie der zumindest abschnittsweise kreisbogenförmige Querschnitt des Trägerelements aufweist. Diese Ausführungsform ermöglicht ein einfaches Gleiten des Trägerelements an dem Führungselement auf der kreisbogenförmigen Bahn.

Bei einer weiteren Ausführungsform ist mit Vorteil vorgesehen, dass die Gehäuseabdeckung an dem Trägerelement angeordnet ist, so dass mit dieser Gehäuseabdeckung das Trägerelement mit dem Kapselhalter handhabbar bzw. in verschiedene Betriebsposition des Vaporizers bringbar ist.

Mit Vorteil ist am Trägerelement eine Führungsnase vorgesehen und eine dazu passende Ausnehmung am Führungselement, wobei diese Führungsnase und die Ausnehmung derart eine Wirkverbindung eingehen, dass die kreisbogenförmige Bewegung des Trägerelements hierdurch geführt durchführbar ist.

Besonders vorteilhaft ist bei einer weiteren Ausführungsform der Offenbarung vorgesehen, dass an dem Führungselement mittels einer Schwenkachse ein Rastelement angeordnet ist, welches mit einer an dem Trägerelement angeordneten Führungsnut in einer Wirkverbindung steht; hierfür ist das Rastelement zwischen einem ersten Anlenkpunkt am Führungselement und einem zweiten Anlenkpunkt am Rastelement mit einer Rückstellfeder wirkverbunden, welche bei der Bedienung des Vaporizers das Rastelement entsprechend verschwenkt.

Bei einer Ausführungsform der Offenbarung sind mit Vorteil zwischen dem Trägerelement und dem Führungselement mindestens ein Führungsstift und eine Öffnungsfeder angeordnet sind. Dabei kann die Öffnungsfeder konzentrisch an dem mindestens einen Führungsstift derart angeordnet sein, dass durch diese Öffnungsfeder die Öffnungsbewegung des Trägerelements bewirkt werden kann.

Ferner ist es von Vorteil, wenn am Führungselement eine Verriegelungsfeder angeordnet ist, die mit einer Führungsnut -angeordnet an dem Trägerelement- eine Wirkverbindung aufweist.

Ohne den vorliegenden grundsätzlichen Offenbarungsgedanken zu verlassen ist es selbstverständlich denkbar, dass zum Ausfahren des Trägerelements aus dem Vaporizer dieses Trägerelement mittels eines hier nicht näher gezeigten bzw. nicht beschriebenen Druckzylinders, einer Spannfeder oder einer Druckfeder auf der kreisbogenförmigen Bewegungsbahn unterstützt wird.

### Kurze Beschreibung der Zeichnungen

Im Folgenden wird die Offenbarung anhand von Figuren beispielhaft erläutert. Gleiche Gegenstände sind in den Figuren grundsätzlich mit gleichen Bezugszeichen versehen. An dieser Stelle wird darauf hingewiesen, dass die Figuren keine beschränkende Wirkung auf den vorliegenden Erfindungsgegenstands als solches haben, sondern lediglich exemplarische Ausführungsformen des Erfindungsgedankens darstellen. Es zeigen rein schematisch die
Fig. 1a, b in einer perspektivischen Seitenansicht einen Vaporizer zur Verdampfung eines Inhalationsmediums gemäss der Offenbarung mit einem aus diesem Vaporizer ausfahrbaren Kapselhalter;
Fig. 2a, b den offenbarungsgemässen Vaporizer in einer Seitenansicht mit einem (a) angedeutetem Teilschnitt A - A' bzw. (b) diesen Teilschnitt A - A' eines Gehäuses des Vaporizers;
Fig. 3a - d den offenbarungsgemässen Vaporizer in einer bildlichen Abfolge der Betriebspositionen (a) geschlossen, (b) zum Öffnen betätigt, (c) offen und (d) zum Schliessen betätigt

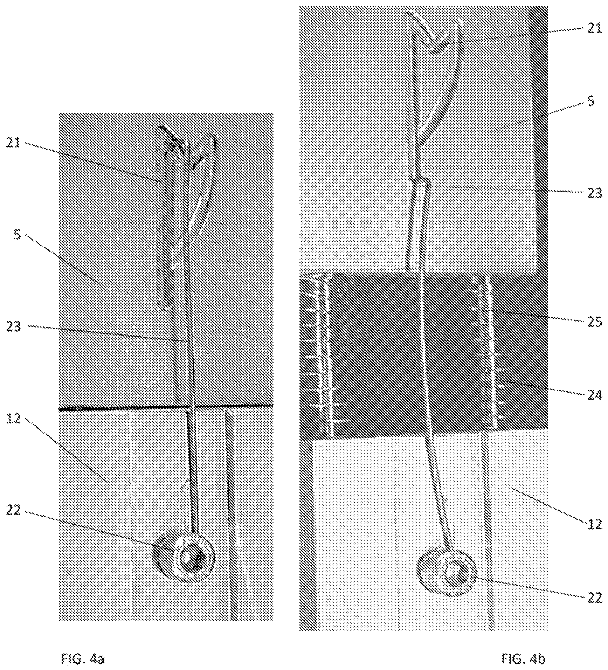

Fig 4a, b eine Ausführungsform des offenbarungsgemässen Vaporizers, mit einer Öffnungsfeder und mindestens einem Führungsstift.

### Wege zur Ausführung der Offenbarung

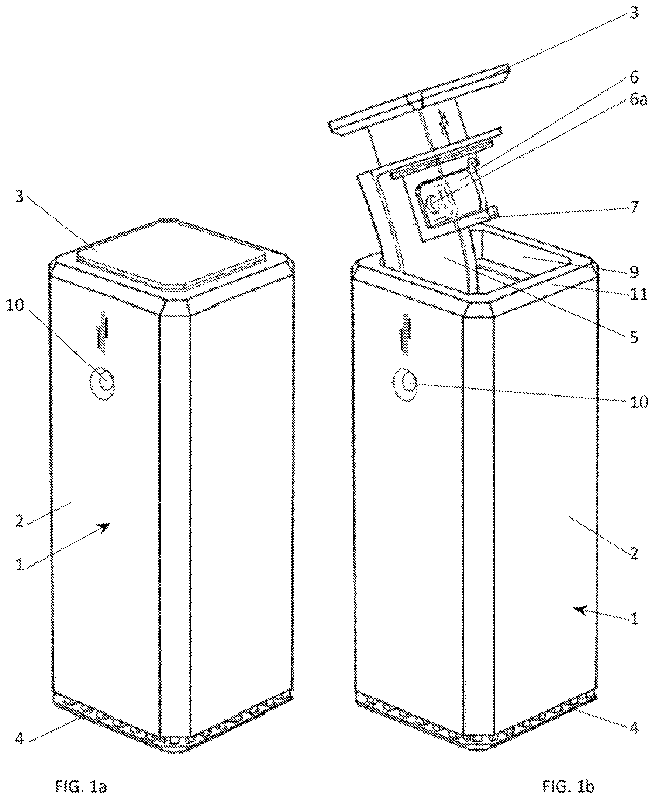

Fig. 1a, b zeigen in einer perspektivischen Seitenansicht den erfindungsgemässen Vaporizer 1 zum einen geschlossen (a) und zum anderen zwecks Befüllung geöffnet (b). Der Vaporizer 1 umfasst -wie Fig. 1a zeigt- ein Gehäuse 2 mit einer Zuluftöffnung 4 im unteren Bereich und einem Dampfauslass 10 an einer Seitenwand des Vaporizers 1. Im oberen Bereich ist der Vaporizer 1 mit einer Gehäuseabdeckung 3 verschlossen. In Fig. 1b ist der Vaporizer 1 in einer Betriebsposition gezeigt, bei welcher ein Kapselhalter 7 aus dem Gehäuse 2 zwecks Bestückung mit einer Kapsel 6 herausragt, die mit einem Inhalationsmedium befüllt ist. In dieser perspektivischen Ansicht ist eine Kapselöffnung 6a der Kapsel 6 erkennbar; eine weitere Kapselöffnung ist in dieser Perspektive nicht dargestellt. Der Kapselhalter 7 ist an einem Trägerelement 5 angeordnet, welches es ermöglicht, den Kapselhalter 7 entlang einer Kreisbogenbahn -die im Folgenden noch näher beleuchtet werden wirdderart aus dem Vaporizer 1 herauszuschwenken, dass der Kapselhalter 7 möglichst einfach und rasch mit einer Kapsel 6 bestückbar ist; hierfür ragt der Kapselhalter 7 aus einer Gehäuseöffnung 9 eines Gehäuserahmens 11 vollständig heraus.

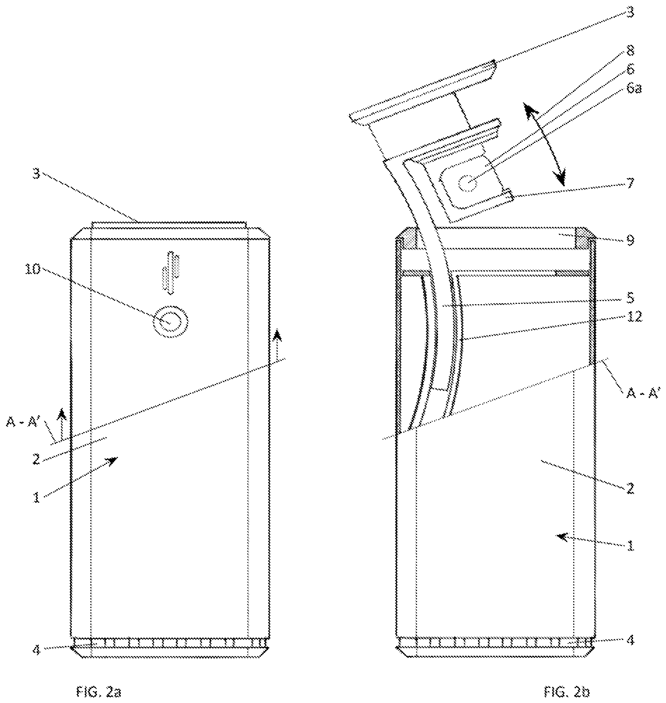

In Fig. 2a, b ist der offenbarungsgemässe Vaporizer 1 in einer Seitenansicht mit einem in Fig. 2a angedeutetem Teilschnitt A - A' am Gehäuse gezeigt bzw. mit diesem Teilschnitt A - A' am Gehäuse des Vaporizers in Fig. 2b; in dieser Fig. 2b ist mit 8 die Bewegungsrichtung des Trägerelements 5 und des daran angeordneten Kapselhalters 7 beim Öffnen bzw. beim Schliessen gezeigt. Das Trägerelement 5 weist hierfür einen kreisbogenförmigen Querschnitt auf und ist an dem ebenfalls mit kreisbogenförmigem Querschnitt versehenen Führungselement 12 verschiebbar angeordnet, wie mit dem Doppelpfeil und den damit angedeuteten Bewegungsrichtungen 8 gezeigt.

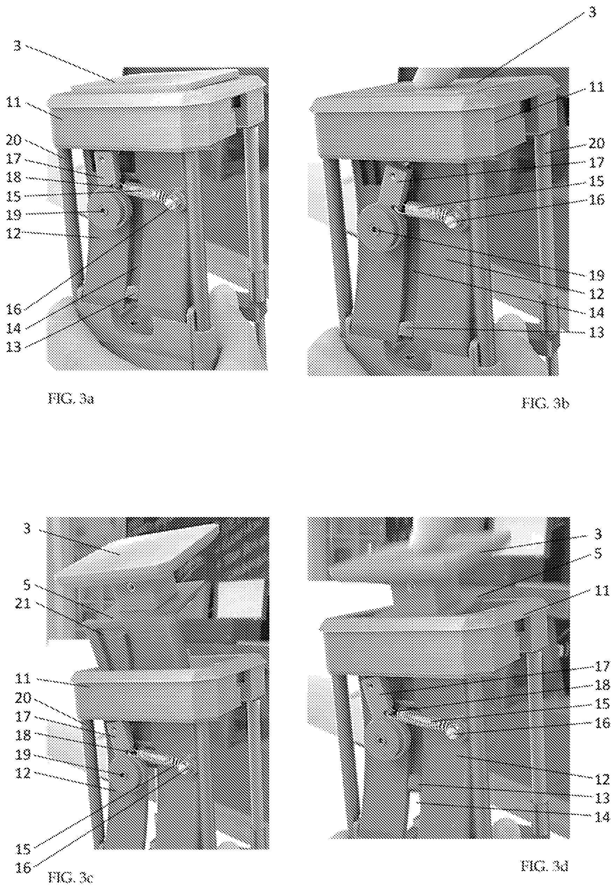

Die Fig. 3a - d offenbaren den offenbarungsgemässen Vaporizer 1 ohne dessen Gehäuse (2, siehe Fig. 1a,b bzw. 2a,b) in einer bildlichen Abfolge von wesentlichen Betriebspositionen, namentlich (a) geschlossen, (b) zum Öffnen betätigt, (c) offen und (d) zum Schliessen betätigt. Konstruktiv umfasst der Vaporizer 1 im Innern vier Gerätestreben 20, die eine statische Grundkonstruktion ergeben, an welcher im Wesentlichen die notwendigen Funktionseinheiten des Vaporizers 1 angeordnet sind.

In der geschlossenen Position siehe Fig. 3a, ist eine Ausnehmung 16 am Führungselement 12 für eine Führungsnase 13 am Trägerelement 5 gezeigt, wobei mittels dieser Führungsnase 13 beim Öffnen des Vaporizers 1 bzw. beim Schliessen desselben eine geführte Bewegung erzwungen wird. Zudem dient diese Führungsnase 13 beim Herausfahren des Trägerelements 5 aus dem Gehäuse 2 des Vaporizers 1 dem Betätigen eines Rastelements 17. Dieses Rastelement 17 ist an dem Führungselement 12 mittels einer Schwenkachse 19 angeordnet, wobei während der Betätigung des Trägerelements 5 dieses Rastelement 17 in einer Führungsnut 21 am Trägerelement 5 geführt wird. Ist dieses Trägerelement 5 zwecks Befüllung aus dem Vaporizer 1 herausgefahren, betätigt bzw. verschwenkt die Führungsnase 13 das Rastelement 17 derart in der Führungsnut 21, dass der Vaporizer 1 in einem nächsten Schritt geschlossen werden kann und in dieser geschlossenen Position, siehe Fig. 3a, bis zum nächsten Betätigen verbleibt. Bei Betätigen der Gehäuseabdeckung 3, siehe Fig. 3b, wird das Rastelement 17 mittels einer Rückstellfeder 15 in eine Freigabeposition geschwenkt, und das Führungselement 5 ist aus dem Vaporizer 1 ausfahrbar, siehe Fig. 3c, zwecks Öffnens des Kapselhalters 7 und dem zeitgleichen Auslenken des Rastelements 17 durch die Führungsnase 13. Das Rastelement 17 wird zum einen mittels der Rückstellfeder 15 in eine erste Betriebsposition geschwenkt, siehe Fig. 3b, in welcher sich der Vaporizer 1 öffnen lässt, und zum anderen mittels der Führungsnase 13 in eine zweite Betriebsposition geschwenkt, siehe Fig. 3c, d, in welcher sich der Vaporizer 1 schliessen lässt. In einer weiteren Betriebsposition siehe Fig 3a, verbleibt das Rastelement 17 - gehalten durch die hier nicht sichtbare Führungsnut 21, Fig. 3c- während der Vaporizer 1 verschlossen ist, bis zum nächsten Betätigen der Gehäuseabdeckung 3, Fig. 3b.

Die Rückstellfeder 15 ist in einfacher Weise zum einen an einem Anlenkpunkt 16 des Führungselements 12 angeordnet und zum anderen an einem weiteren Anlenkpunkt 18 des Rastelements 17.

Fig. 3d zeigt eine Betriebsposition des Trägerelements 5 beim Schliessen des Vaporizers 1 mittels Betätigens der Gehäuseabdeckung 3. Während des Schliessens verbleibt das Rastelement 17 -geführt durch die Führungsnut 21- in der ausgelenkten Betriebsposition, wie auch in Fig. 3c zeigt.

Bei einer Ausführungsform der Offenbarung sind zwischen dem Trägerelement 5 und dem Führungselement 12 mindestens ein Führungsstift 24 und eine Öffnungsfeder 25 vorgesehen. Dabei kann die Öffnungsfeder 25 konzentrisch an dem mindestens einen Führungsstift 14 derart angeordnet sein, dass durch diese Öffnungsfeder 25 die Öffnungsbewegung des Trägerelements bewirkt werden kann.

Ferner ist es von Vorteil, wenn am Führungselement 12 eine Verriegelungsfeder 23 an einem Befestigungsmittel 22 -vorliegend eine Schraubeangeordnet ist, wobei diese Verriegelungsfeder 23 mit einer Führungsnut 21 - angeordnet an dem Trägerelement 5- eine Wirkverbindung aufweist.

Gegenüber herkömmlichen Vaporizern ist der Vaporizer gemäss vorliegender Offenbarung einfacher bedienbar und muss zum Befüllen mit einer Kapsel nicht vorgängig zerlegt, befüllt und wieder zusammengesetzt werden, was eine raschere und auch sicherere Handhabung des Vaporizers jederzeit gewährleistet.

### Bezugszeichenliste

- 1: Vaporizer
- 2: Gehäuse
- 3: Gehäuseabdeckung
- 4: Zuluftöffnung
- 5: Trägerelement
- 6: Kapsel
- 6a: Kapselöffnung
- 7: Kapselhalter
- 8: Bewegungsrichtungen von 5
- 9: Gehäuseöffnung
- 10: Dampfauslass
- 11: Gehäuserahmen
- 12: Führungselement
- 13: Führungsnase
- 14: Ausnehmung an 12 für 13
- 15: Rückstellfeder
- 16: Anlenkpunkt an 12 für 15
- 17: Rastelement
- 18: Anlenkpunkt an 17 für 15
- 19: Schwenkachse an 12 für 17
- 20: Gerätestrebe für Innenkonstruktion von 1
- 21: Führungsnut an 5 für 17
- 22: Befestigungsmittel, Schraube
- 23: Verriegelungsfeder
- 24: Führungsstift
- 25: Öffnungsfeder

## Patentansprüche

1. Vaporizer (1) zur Verdampfung eines Inhalationsmediums, umfassend ein Gehäuse (2) mit einer Gehäuseabdeckung (3) in einem oberen Bereich des Gehäuses (2), ferner umfassend eine Zuluftöffnung (4) in einem unteren Bereich des Gehäuses (2) und einen Dampfauslass (10), wobei der Vaporizer (1) ein Trägerelement (5) mit einem daran angeordneten Kapselhalter (7) aufweist, wobei dieses Trägerelement (5) zumindest abschnittsweise einen kreisbogenförmigen Querschnitt aufweist und wobei der Kapselhalter (7) mit diesem Trägerelement (5) an einem Führungselement (12) entlang einer kreisbogenförmigen Bewegungsrichtung (8) verschiebbar angeordnet ist und der Kapselhalter (7) in einer geöffneten Position des Trägerelements (5) bedienbar respektive mit dem Inhalationsmedium befüllbar aus dem Vaporizer (1) herausragt, **dadurch gekennzeichnet, dass** an dem Führungselement (12) mittels einer Schwenkachse (19) ein Rastelement (17) angeordnet ist, welches mit einer Führungsnut (21) - angeordnet an dem Trägerelement (5)- in einer Wirkverbindung steht.

2. Vaporizer (1) nach Anspruch 1, **dadurch gekennzeichnet, dass** das Führungselement (12) ebenfalls einen zumindest abschnittsweise kreisbogenförmigen Querschnitt aufweist, wobei der zumindest abschnittsweise kreisbogenförmige Querschnitt des Führungselements (12) einen identischen Radius wie der zumindest abschnittsweise kreisbogenförmige Querschnitt des Trägerelements (5) aufweist.

3. Vaporizer (1) nach einem der Ansprüche 1 oder 2, **dadurch gekennzeichnet, dass** die Gehäuseabdeckung (3) an dem Trägerelement (5) angeordnet ist, wobei mit dieser Gehäuseabdeckung (3) das Trägerelement (5) mit dem Kapselhalter (7) in eine Verschlussposition des Vaporizers (7) bringbar ist.

4. Vaporizer (1) nach einem der Ansprüche 1 bis 3, **dadurch gekennzeichnet, dass** das Trägerelement (5) eine Führungsnase (13) aufweist und das Führungselement (12) eine Ausnehmung (14), wobei diese Führungsnase (13) und die Ausnehmung (14) in einer Wirkverbindung angeordnet sind.

5. Vaporizer (1) nach einem der Ansprüche 1 bis 4, **dadurch gekennzeichnet, dass** zwischen einem Anlenkpunkt (16) am Führungselement (12) und einem Anlenkpunkt (18) am Rastelement (17) eine der Wirkverbindung dienende Rückstellfeder (15) angeordnet ist.

6. Vaporizer (1) nach einem der Ansprüche 1 bis 5, **dadurch gekennzeichnet, dass** zwischen dem Trägerelement (5) und dem Führungselement (12) mindestens ein Führungsstift (24) und eine Öffnungsfeder (25) angeordnet sind.

7. Vaporizer (1) nach einem der Ansprüche 1 bis 6, **dadurch gekennzeichnet, dass** an einem Befestigungsmittel (22) am Führungselement (12) eine Verriegelungsfeder (23) angeordnet ist, die mit einer Führungsnut (21) -angeordnet an dem Trägerelement (5)- eine Wirkverbindung aufweist.

## Claims

1. Vaporizer (1) for vaporizing an inhalation medium, comprising a housing (2) with a housing cover (3) in an upper region of the housing (2), further comprising an air inlet opening (4) in a lower region of the housing (2) and a vapor outlet (10), wherein the vaporizer (1) has a carrier element (5) with a capsule holder (7) arranged thereon, wherein said carrier element (5) has, at least in sections, a circular-arc-shaped cross-section, and wherein the capsule holder (7), together with said carrier element (5), is arranged on a guide element (12) so as to be displaceable along a circular-arc-shaped direction of movement (8), and the capsule holder (7), in an open position of the carrier element (5), protrudes from the vaporizer (1) so as to be operable or fillable with the inhalation medium, **characterized in that** a latching element (17) is arranged on the guide element (12) by means of a pivot axis (19), which latching element (17) is operatively connected to a guide groove (21) arranged on the carrier element (5).

2. Vaporizer (1) according to claim 1, **characterized in that** the guide element (12) likewise has a cross-section which is circular-arc-shaped at least in sections, wherein the cross-section of the guide element (12) which is circular-arc-shaped at least in sections has a radius identical to that of the cross-section of the carrier element (5) which is circular-arc-shaped at least in sections.

3. Vaporizer (1) according to claim 1 or 2, **characterized in that** the housing cover (3) is arranged on the carrier element (5), wherein, by means of said housing cover (3), the carrier element (5) with the capsule holder (7) can be brought into a closed position of the vaporizer (1).

4. Vaporizer (1) according to any one of claims 1 to 3, **characterized in that** the carrier element (5) has a guide lug (13) and the guide element (12) has a recess (14), wherein said guide lug (13) and the recess (14) are arranged in operative connection with one another.

5. Vaporizer (1) according to any one of claims 1 to 4, **characterized in that** a return spring (15) serving the operative connection is arranged between an articulation point (16) on the guide element (12) and an articulation point (18) on the latching element (17).

6. Vaporizer (1) according to any one of claims 1 to 5, **characterized in that** at least one guide pin (24) and an opening spring (25) are arranged between the carrier element (5) and the guide element (12).

7. Vaporizer (1) according to any one of claims 1 to 6, **characterized in that** a locking spring (23) is arranged on a fastening means (22) on the guide element (12), which locking spring (23) is operatively connected to a guide groove (21) arranged on the carrier element (5).

## Revendications

1. Vaporisateur (1) destiné à vaporiser un milieu d'inhalation, comprenant un boîtier (2) avec un couvercle de boîtier (3) dans une zone supérieure du boîtier (2), comprenant en outre une ouverture d'entrée d'air (4) dans une zone inférieure du boîtier (2) et une sortie de vapeur (10), le vaporisateur (1) présentant un élément porteur (5) avec un porte-capsule (7) disposé sur celui-ci, cet élément porteur (5) présentant, au moins par tronçons, une section transversale en arc de cercle, et le porte-capsule (7) étant disposé, avec cet élément porteur (5), sur un élément de guidage (12) de manière à pouvoir coulisser le long d'une direction de déplacement en arc de cercle (8), et le porte-capsule (7), dans une position ouverte de l'élément porteur (5), faisant saillie hors du vaporisateur (1) de manière à pouvoir être manipulé ou rempli avec le milieu d'inhalation, **caractérisé en ce qu'**un élément d'encliquetage (17) est disposé sur l'élément de guidage (12) au moyen d'un axe de pivotement (19), lequel élément d'encliquetage (17) est en liaison fonctionnelle avec une rainure de guidage (21) disposée sur l'élément porteur (5).

2. Vaporisateur (1) selon la revendication 1, **caractérisé en ce que** l'élément de guidage (12) présente également une section transversale en arc de cercle au moins par tronçons, la section transversale en arc de cercle au moins par tronçons de l'élément de guidage (12) présentant un rayon identique à celui de la section transversale en arc de cercle au moins par tronçons de l'élément porteur (5).

3. Vaporisateur (1) selon la revendication 1 ou 2, **caractérisé en ce que** le couvercle de boîtier (3) est disposé sur l'élément porteur (5), l'élément porteur (5) avec le porte-capsule (7) pouvant être amené, au moyen de ce couvercle de boîtier (3), dans une position de fermeture du vaporisateur (1).

4. Vaporisateur (1) selon l'une quelconque des revendications 1 à 3, **caractérisé en ce que** l'élément porteur (5) présente un ergot de guidage (13) et l'élément de guidage (12) présente un évidement (14), cet ergot de guidage (13) et l'évidement (14) étant disposés en liaison fonctionnelle l'un avec l'autre.

5. Vaporisateur (1) selon l'une quelconque des revendications 1 à 4, **caractérisé en ce qu'**un ressort de rappel (15) servant à la liaison fonctionnelle est disposé entre un point d'articulation (16) sur l'élément de guidage (12) et un point d'articulation (18) sur l'élément d'encliquetage (17).

6. Vaporisateur (1) selon l'une quelconque des revendications 1 à 5, **caractérisé en ce qu'**au moins une broche de guidage (24) et un ressort d'ouverture (25) sont disposés entre l'élément porteur (5) et l'élément de guidage (12).

7. Vaporisateur (1) selon l'une quelconque des revendications 1 à 6, **caractérisé en ce qu'**un ressort de verrouillage (23) est disposé sur un moyen de fixation (22) sur l'élément de guidage (12), lequel ressort de verrouillage (23) est en liaison fonctionnelle avec une rainure de guidage (21) disposée sur l'élément porteur (5).
